(19) Europäisches Patentamt
European Patent Office
Office européen des brevets

(11) **EP 4 674 347 A1**

(12) **EUROPEAN PATENT APPLICATION**

(43) Date of publication:
**07.01.2026 Bulletin 2026/02**

(21) Application number: **24386080.6**

(22) Date of filing: **04.07.2024**

(51) International Patent Classification (IPC):
**A61B 5/282** (2021.01)    **A61B 5/00** (2006.01)

(52) Cooperative Patent Classification (CPC):
**A61B 5/282; A61B 5/6804;** A61B 5/291;
A61B 5/296; A61B 2562/125

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Cambridge Enterprise Limited
Cambridge, Cambridgeshire CB2 1TN (GB)**

(72) Inventor: **The designation of the inventor has not
yet been filed**

(74) Representative: **J A Kemp LLP
80 Turnmill Street
London EC1M 5QU (GB)**

(54) **WEARABLE ELECTRODE DEVICE AND METHOD OF MANUFACTURING**

(57) The present invention provides a wearable device for placement on the skin of a wearer and configured to receive and/or transmit signals from or to the wearer, the device having a plurality of layers including: a first, textile layer having a first surface and a second surface opposite the first surface and having a plurality of conductive electrodes formed on the first surface and electrically connected through the first layer to the second surface; and a second layer, formed on the first layer, wherein the second layer has a plurality of conductive vias formed therethrough and a plurality of conductive tracks, the conductive vias connecting the electrodes of the first layer to the conductive tracks, wherein the plurality of conductive vias define a first array, wherein each of the plurality of conductive tracks has a contact point distal from the via to which it is connected, the plurality of contact points defining a second array; and wherein the area covered by the second array is smaller than the area covered by the first array.

Figure 1

**Description**

The present invention relates to devices and methods of manufacturing. The invention is of particular, but not exclusive, relevance to wearable devices, for example those configured to detect and monitor physiological signals from a human or animal body.

Electrophysiology, the study of the electrical properties of biological cells and tissues, has long been a cornerstone in both research and clinical settings. By measuring the electrical activity of neurons, muscle cells and other electrically excitable cells, electrophysiology provides invaluable insights into the functioning of the heart, brain, and other organs. Traditional electrophysiological techniques, such as electrocardiograms (ECGs), electromyograms (EMGs) or electroencephalograms (EEGs), have become essential tools in diagnosing and monitoring a variety of conditions, from cardiac arrhythmias to neurological disorders. However, the clinical landscape is witnessing a significant shift towards high-density body surface potential mapping (BSPM). This advanced approach allows for more precise spatial resolution and detailed characterisation of electrical activity, which is crucial for understanding complex physiological phenomena and improving patient outcomes.

Flexible substrate electrode array fabrication techniques employing polymeric substrates such as polyimide (Kapton) are at the forefront of this transition due to their excellent thermal and chemical stability, durability and flexibility. However, despite their advantages, polymeric-substrate-based high-density electrode arrays face significant limitations, particularly in terms of conformability. The rigid nature of these substrates can hinder their ability to closely adapt to the heterogeneous and dynamic surfaces of biological tissues, potentially compromising the accuracy and efficacy of measurements.

In response to these challenges, e-textiles have emerged as a promising alternative fabrication method. E-textiles combine the electrical functionality of traditional sensors with the flexibility and comfort of fabrics, offering a new level of adaptability and integration with the human body. However, current e-textile technologies have two significant limitations that need to be addressed: the limitations on achievable resolution (usually ~1mm) and the integration of reliable electrical connections.

For example, for certain electrode arrays, in order to obtain sufficient resolution from the electrodes, it is desirable that the electrodes form an array covering a relatively large area on the subject. However, connecting such electrodes to processing electronics, whether as part of the wearable device itself, or via a wired or wireless connection normally necessitates individual wires from each electrode, often covering significant distances. This is cumbersome and can lead to devices being impractical outside a clinical setting and/or to reduced user acceptance.

In other electrode arrays it is desirable to have a high density of electrodes in a relatively small area (i.e. with the electrodes close to each other). In such configurations connecting the electrodes to processing electronics can also be challenging due to the high density of the electrodes and the small space available.

Furthermore, many current electrodes dry up over time, which limits their usefulness for long-term recording/monitoring.

There is therefore a need for improved wearable electrode arrays which are also desirably more convenient for the wearer.

It is an aim of the present invention to overcome one or more of these limitations. This could pave the way for a wide range of future applications in both clinical and research settings. These include, without limitation, Holter monitors, ECG, EEG, EMG, mechanical heart conditions, and the measurement of any other electrophysiology signals.

At their broadest, aspects of the present invention provide devices and methods of manufacturing devices, where the devices bring together the inputs from a disparate array of sensors distributed across the desired detection area to a convenient connection configuration having a smaller area.

A first aspect of the present invention provides a wearable device for placement on the skin of a wearer and configured to receive and/or transmit signals from or to the wearer, the device having a plurality of layers including: a first, textile layer having a first surface and a second surface opposite the first surface and having a plurality of conductive electrodes formed on the first surface and electrically connected through the first layer to the second surface; and a second layer, formed on the first layer, wherein the second layer has a plurality of conductive vias formed therethrough and a plurality of conductive tracks, the conductive vias connecting the electrodes of the first layer to the conductive tracks, wherein the plurality of conductive vias define a first array, wherein each of the plurality of conductive tracks has a contact point distal from the via to which it is connected, the plurality of contact points defining a second array; and wherein the area covered by the second array is smaller than the area covered by the first array.

The device of this aspect can enable an array of electrodes to be provided which are positioned in a known relationship to each other. The device can also enable these electrodes to be arranged on a scale which is desirable for the sensing of, for example, physiological signals. The arrangement of the electrodes may also be on a scale suitable for relative discrimination of signals from a wearer, whilst being connected to a common interface which is configured on a (generally much smaller) scale suitable for electronics (for example, for connection to electronic components and/or to a common communications link). This configuration can mean that it is not necessary to have (multiple) individual wires exiting the device which connect to the individual electrodes.

Preferably the first and second layers are flexible so that the device as a whole is flexible. This can permit the device to be worn by a user and to conform to the portion of the wearer's body to which it is applied.

In certain embodiments, the device is part of, or is connected to, other elements which are configured to enable and assist in the application of the device to a wearer and to keep the device in a stable and consistent position on the wearer. These may include stretchable fabric elements, fasteners (such as hook and loop type fasteners) and/or adjustable straps or ties.

Preferably the device is configured to conform to the skin of the wearer.

The textile layer preferably makes the device comfortable for a user to wear against their skin. This can help to provide a device which has better user acceptance than the application of individual electrodes to the skin.

The textile layer is preferably porous in order to allow the electrodes to be formed by printing the conductive material of the electrodes onto one side of the textile material and for the conductive material to pass through and be incorporated into the textile layer without significant sideways spread, such that the electrodes formed on the first and second surfaces of the textile layer exhibit good conductivity across the textile layer and are of approximately similar dimensions (ideally substantially identical sizes) when viewed in the plane of the device. The porosity of the textile layer can also assist in rendering the device as a whole breathable (i.e. such that air and moisture will pass through the device). This can improve comfort and/or wearability for the user.

The textile layer is preferably made from a textile that is resilient to multiple heating processes at over 100 degrees without losing its shape, form or elasticity. This enables the various layers of the device to be formed on the textile layer and cured without damaging the desirable properties which the textile layer brings to the device.

The device may include a printed circuit board electrically connected to a plurality of said contact points. The printed circuit board may contain processing electronics for processing signal inputs from the electrodes. Alternatively or additionally the printed circuit board may provide for communication to an external device, either through a wired or wireless connection. In embodiments which provide for a wireless connection, the device may have an antenna, which may, for example, be formed on or attached to the printed circuit board. The printed circuit board may be flexible.

In certain embodiments the device further includes a third layer formed on the second layer, the conductive tracks and contact points being encapsulated by the third layer such that the conductive tracks are not accessible through the third layer, but the contact points are accessible. This ensures that the conductive elements are sealed within the device and therefore can reduce or eliminate adverse effects from the contact of these elements with the environment. A lamination approach such as this can also be effective to overcome wiring scalability limitations for example by permitting multiple layers of tracks and vias in order to incorporate the connection of a large number of electrodes to corresponding contact points.

The device may also include a eutectogel coating on the electrodes on the first side of the textile layer. Many current electrodes dry up over time, which limits their usefulness for long-term recording/monitoring, but the provision of the eutectogel coating can reduce or prevent this.

In certain embodiments the vias, tracks and contact points may be formed of silver (and/or a silver-based substance such as silver nanoparticle inks), graphite, copper, gold or any other commonly employed metals or conductive polymers (such as PEDOT:PSS).

The device may have any number of electrodes (and corresponding numbers of vias, tracks and contact points). In some embodiments, two electrodes are sufficient for useful physiological monitoring. However, in other embodiments, there are at least 6, at least 10, at least 15 or at least 20 electrodes, vias, tracks and contact points.

The electrodes may be arranged in a regular pattern. The regular pattern may be such that the electrodes are at substantially the same distance from each neighbouring electrode when viewed in the plane of the electrodes. This pattern may be polyhedral, for example hexagonal, which is a configuration which provides for a compact but regular distribution of the electrodes.

The area covered by the first array may vary considerably depending on the intended application (including the type of animal to which the device is intended to be applied). Typically the area covered by the first array is at least 1 cm x 1 cm (i.e. 1 cm$^2$). In certain embodiments the area covered by the first array is at least 25 cm$^2$, at least 100 cm$^2$, or at least 1000 cm$^2$. For application to larger animal subjects, the area covered by the first array may be at least 1 m$^2$, at least 10 m$^2$ or at least 100 m$^2$.

The area covered by the second array is typically of the size of known printed circuit boards. Typically this area will be at least 0.25 cm$^2$ (0.5 cm x 0.5 cm) and at most 2500 cm$^2$ (25 cm x 25 cm). In some embodiments the area covered by the second array is no more than 1000 cm$^2$, no more than 500 cm$^2$, no more than 100 cm$^2$ or no more than 25 cm$^2$.

The ratio of the area covered by the first array to the area covered by the second array may be 2:1, preferably at least 5:1, more preferably at least 10:1. In some embodiments the ratio may be considerably larger, for example at least 100:1 or at least 1000:1.

The devices of this aspect can have mm-size, low-impedance conducting polymer coated electrodes. Such a device can potentially achieve unprecedented spatial resolution on BSPM and other electrophysiological measurements.

The devices can also have long-term recording capabilities as the electrodes will not dry up in the manner of known polymer electrodes.

Prototype arrays with optimally chosen conducting polymer formulation and geometrical parameters have been fabricated and measured on polymeric substrates to demonstrate the detection of physiological signals (including ECG, EMG, EEG, etc.) with comparable signal-to-noise ratio (SNR) to larger area commercial Ag-Ag chloride (Ag/AgCl) electrodes. Alternatively, for similar area arrays, improved SNR can be achieved.

Furthermore, the devices according to this aspect can provide high-density electrode arrays which allow additional spatial information to be obtained from physiological signals.

The device of the above aspect may include some, all or none of the above-described optional and preferred features in any combination.

A second aspect of the present invention provides a method of fabricating a wearable device, the method including the steps of: forming a plurality of conductive electrodes on a textile layer, wherein the electrodes extend from a first side of the textile layer to a second, opposite, side; forming a second, insulating layer on the second side of the textile layer, overlaying the electrodes; forming through-holes in the second layer at the locations of the electrodes; applying conductive material to the through-holes to form conductive vias connected to the electrodes, the plurality of conductive vias defining a first array; and forming a plurality of tracks on the second layer, each track connecting one of the conductive vias to a contact point distal from the conductive via, the plurality of contact points forming a second array, wherein the area covered by the second array is smaller than the area covered by the first array.

The method preferably further includes the step of forming a third layer over the tracks such that the conductive tracks are encapsulated between the second and third layers, but the contact points are accessible through the third layer. This ensures that the conductive elements are sealed within the device and therefore can reduce or eliminate adverse effects from the contact of these elements with the environment. A lamination approach such as this can also be effective to overcome wiring scalability limitations for example by permitting multiple layers of tracks and vias in order to incorporate the connection of a large number of electrodes to corresponding contact points.

The method preferably further includes the step of connecting a printed circuit board to the contact points.

The step of forming the electrodes may use blade-coating or screen-printing. Blade-coating has been found to be particularly effective in causing the applied conductive material to pass through the textile layer in order to be incorporated into the textile layer without significant sideways spread, such that the electrodes formed on the first and second surfaces of the textile layer exhibit good conductivity across the textile layer and are of approximately similar dimensions (ideally substantially identical sizes) when viewed in the plane of the device.

The method may include the further step of forming a eutectogel coating on the electrodes on the first side of the textile layer. Many current electrodes dry up over time, which limits their usefulness for long-term recording/monitoring, but the provision of the eutectogel coating can reduce or prevent this.

The method of this aspect may include some, all or none of the above-described optional and preferred features in any combination.

Preferably the method is a method of making the device of the above first aspect, but need not be. Where the method is a method of making the device of the above first aspect, it may include some, all or none of the above-described optional and preferred features of that aspect in any combination.

Unless indicated otherwise, any of the features (including the optional or preferred features) described in relation to one of the above aspects are equally applicable in combination with the devices, systems and methods of any of the other above-described aspects.

The invention is described below, by way of example, with reference to the accompanying figures in which:

Figure 1 shows a cross-sectional view through a device according to an embodiment of the invention, along with top views of the device at the layers indicated;

Figure 2 shows schematic views of various parts of a device according to an embodiment of the present invention; and

Figure 3 shows views of a finished device according to an embodiment of the present invention.

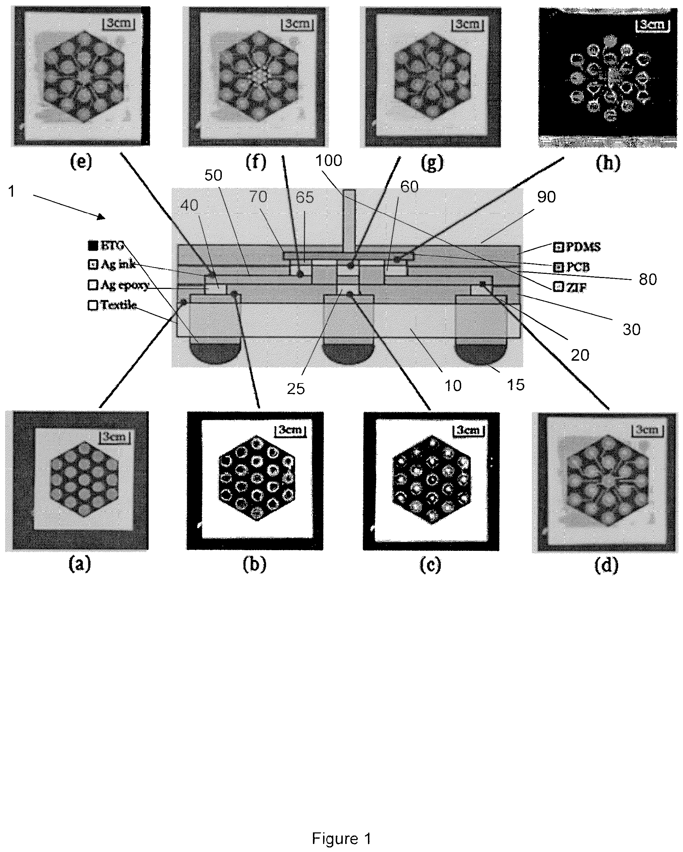

Figure 1 shows a cross-sectional view through a device according to an embodiment of the present invention, and is also used to illustrate the method of manufacturing such a device according to a further embodiment of the present invention.

The electrode layer 20 is formed by means of a double-sided, Ag nanoparticle blade-coating technique. Initially, the textile layer 10 was pre-stretched against a flat surface and, whilst held in position, patterned with an adhesive mask formed by double-sided tape Causing tension on fabrics before deposition has been shown to minimise the absorption coefficient of textile fibres. 50% pre-stretching over the original length of the textile was thus implemented, causing a significant decrease in sideways spread of the Ag ink, the formation of a uniform conducting saturation layer and an increase in shape resolution. The blade-coating causes the Ag nanoparticle ink to penetrate the textile layer 10 so that contact electrodes are formed which extends through the textile layer from the body-contacting side (the lower side in Figure 1) to the contacts side (the upper side in Figure 1).

The double-sided tape mask is then removed from one of the textile sides and a new mask from the same material is attached to define the edges of the hexagonal profile of the device 1 when viewed from above. Non-cross-linked polydimethylsiloxane (PDMS) is poured over the electrodes and blade-coated with a squeegee to remove excess, generating a uniform insulation layer 30 moulded by the double-sided tape mask, see Figure 1(a). The PDMS layer 30 is subsequently cross-linked in an oven (105°C for one hour). The PDMS layer 30 penetrates into the textile layer 10 and thus bonds the device together.

In order to prevent lack of insulation at the edges of the Ag layer, a second double-sided tape mask with the same shape is attached over the first one and a second deposition of PDMS is performed. Once cross-linked, the PDMS is rastered in circular regions over the electrode areas by means of a laser cutter, see Figure 1(b).

A mask of silicon-based tape is then generated with the shape of the rastered holes and Ag epoxy is applied by means of a small swab to form vias 40. The silicon-based tape mask is removed and the epoxy is cured in the oven (105°C for 30 minutes), see Figure 1(c).

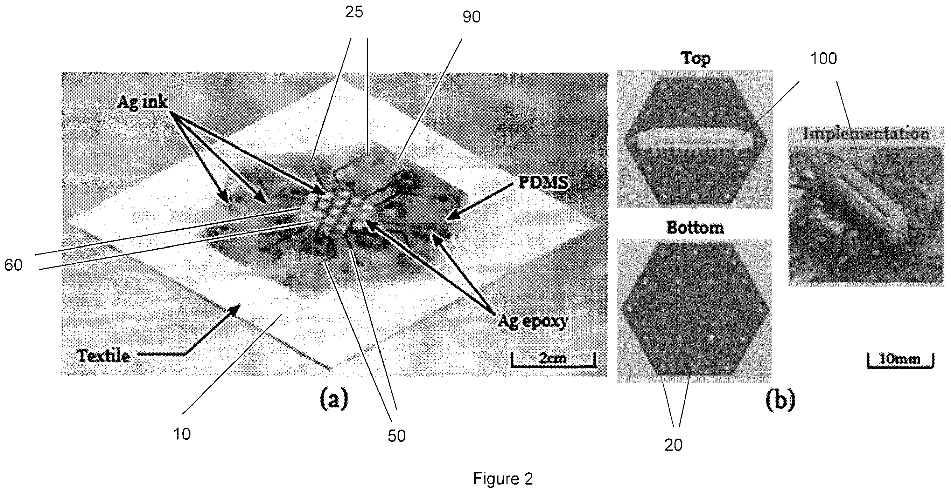

A second mask of silicon-based tape is produced with the track layer pattern (for example as shown in Figure 2(a)) and adhered to the PDMS substrate 30. Ag nanoparticle paste is then blade-coated on the PDMS surface 30 with a squeegee to form the tracks 50 connected to the vias 40. The mask is removed and the Ag is cured in the oven (105°C for 30 minutes), see Figure 1(d).

In order to prevent the cured Ag tracks 50 from peeling from the PDMS substrate 30, non-cross-linked PDMS 80 is carefully applied to the tracks with a brush, leaving the areas of contact for the pads in the centre of the structure uncovered, see Figure 1(e). The PDMS is cross-linked in the oven (105°C for one hour) and a third silicon-based tape mask with the central pads pattern is attached to this PDMS layer 80. Ag epoxy is applied again by means of a small swab to form vias 60 and cured (105°C for 30 minutes) after removal of the mask, as shown in Figure 1(f).

The resulting structure is covered with a fourth silicon-based mask with the same central pads pattern and Ag nanoparticle paste 65 is blade-coated with a squeegee over the Ag epoxy contact pads 60, see Figure 1(g). While the Ag is uncured, the custom PCB 70 which is used to interface the electrodes 20 with external electronics is placed over the Ag paste 65 by aligning the bottom pads of the PCB 70 with the top pads of the device 1. The Ag paste is then cured in an oven (105°C for 30 minutes) and a final double-sided tape mask with the shape of the device contour is attached over the previous two to allow for a final non cross-linked PDMS deposition 90 which both seals the PCB against the substrate and increases the robustness to bending of the Ag tracks.

The PDMS is cross-linked in the oven (105°C for one hour), followed by the removal of the double-sided tape masks used for contour, see Figure 1(h). Finally, a eutectogel (ETG) coating 15 is deposited at the other side of the textile layer 10 and the tape mask on that side is removed after ETG cool-down.

To improve the integration of the laminated layers on the textile and the overall robustness of the device 1, a silicone rubber, such as PDMS, was applied in a layer 30 to surround and contain the conductive tracks 50. Applying PDMS to textile in its non-cross-linked, liquid state has been shown to allow for the creation of dyeing patterns embedded within a fabric. A similar approach was implemented to insert further track lamination layers over the blade-coated Ag electrodes 20, as shown in Figure 1 and Figure 2a.

The device 1 achieves multi-layer lamination by consecutively alternating between PDMS insulation 30, 80, 90 and Ag nanoparticle paste wiring 20, 50, 65. The tracks connecting to electrodes further away from the external wire interface run above those of closer electrodes by means of intermediate insulation layers.

In order to retain a single interfacing plane, the contact points for the deeper tracks pierce through these layers by means of vias 25, 60 implemented with depositions of Ag epoxy (8330S-21G, MGChemicals). The external routing is performed by means of a custom-made interfacing printed circuit board (PCB) 70 inserted at the top of the upper PDMS layer, as shown in Figure 2(b). The bottom side of the PCB 70 consists of 19 via holes placed at specific locations designed to match those fabricated onto the device. The top side connects those holes to the pads of a surface-mounted device footprint corresponding to a standard vertical, 20 ways, 0.5mm pitch ZIF connector 100. The PCB 70 is connected to the device and sealed with an additional layer of PDMS 90.

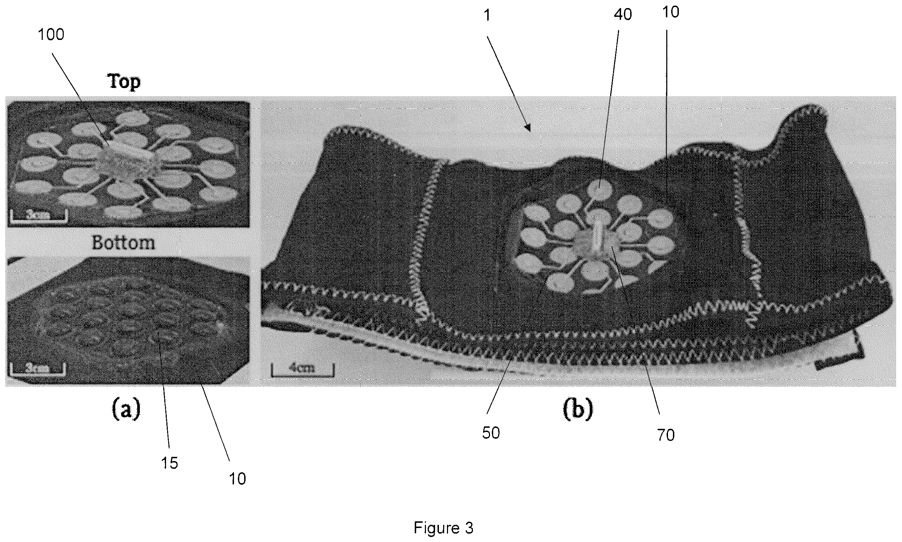

The final device 1 manufactured using the above methods is shown in Figure 3. Figure 3(a) shows top and bottom views of the device 1 and illustrates the relative size of the PCB 70 and the pattern of electrodes 20. Figure 3(b) shows the device 1 incorporated into a textile strap which is sewn to the textile layer 10 of the fabricated device. Given that the PCB 70 allows for any footprint design to be included, it is possible to minimise the number of external wires required to interface with higher density modules.

The adhesion of the array to the body can be tuned for different individuals by employing a hook and loop fastening structure at the edge of the textile substrate 10. The use of a hook and loop fastening system is not only convenient for fitting the device to different subjects and/or to different portions of the subject anatomy, but also to avoid removal of the ETG coating material on the electrodes through shear stress.

*External wiring scalability*

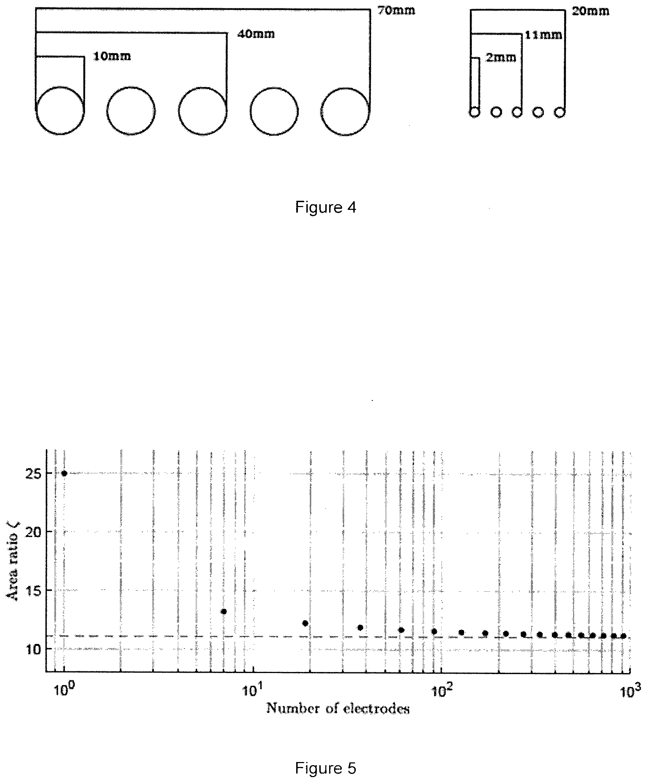

Starting from the design specifications of both electrode arrays and PCB pads it is possible to determine the rate of size increase of the PCB connector area with respect to the electrode array area. The increase in the length of the main axis in the hexagonal structure of both electrodes and pads follows a linear relationship. Figure 4 shows a schematic and simplified example of electrode and connector spacings. Using these values and the general linear equation y = mx + n,

PADS

$$11 = 2m + n \qquad 20 = 3m + n$$

$$\Rightarrow n = 11 - 2m \qquad 20 = m + 11$$

$$\Rightarrow m = 9, n = -7$$

ELECTRODES

$$40 = 2m + n \qquad 70 = 3m + n$$

$$\Rightarrow n = 40 - 2m \qquad 70 = m + 40$$

$$\Rightarrow m = 30, n = -20$$

The ratio between electrode and pad length increase is given by,

$$\xi = \frac{30x - 20}{9x - 7}$$

The limit as x reaches infinity is given by L'Hôpital's rule as

$$\lim_{x \to \infty} (\xi) = \frac{30}{9} = 3.\hat{3}$$

which indicates the length of the long axis of the electrodes tends to be three times larger than that of the PCB pads, regard less of the number of electrodes.

The area ratio between electrodes and pads can be obtained from the definition of the area of a hexagon,

$$A = \frac{3\sqrt{3}}{8} D^2$$

where D represents the long axis of the hexagon. Plugging in the expressions for D calculated for electrodes and pads yields the following expression,

$$\zeta = \left( \frac{30x - 20}{9x - 7} \right)^2$$

Applying L'Hôpital's rule yields

$$\lim_{x \to \infty} (\zeta) = \frac{900}{81} = 11.\hat{1}$$

which indicates the area covered by the PCB pads remains approximately 11 times smaller than that of the electrodes, regardless of the number of electrodes. The progression of the ratio between areas as a function of number of electrodes is depicted in Figure 5. This result demonstrates the external wiring scalability of the proposed e-textile, multi-layer lamination high-density electrode array fabrication process.

The forgoing description is exemplary in nature only, and the skilled person will understand that changes and variations on the disclosed embodiments are possible within the scope of the claims. The claims define the invention.

**Claims**

1. A wearable device for placement on the skin of a wearer and configured to receive and/or transmit signals from or to the wearer, the device having a plurality of layers including:

   a first, textile layer having a first surface and a second surface opposite the first surface and having a plurality of conductive electrodes formed on the first surface and electrically connected through the first layer to the second surface; and
   a second layer, formed on the first layer, wherein the second layer has a plurality of conductive vias formed therethrough and a plurality of conductive tracks, the conductive vias connecting the electrodes of the first layer to the conductive tracks, wherein the plurality of conductive vias define a first array,

   wherein each of the plurality of conductive tracks has a contact point distal from the via to which it is connected, the plurality of contact points defining a second array; and wherein the area covered by the second array is smaller than the area covered by the first array.

2. The device of claim 1 wherein the first layer is flexible.

3. The device of claim 2 wherein the device is configured to conform to the skin of the wearer.

4. The device of any preceding claim wherein the second layer is flexible.

**5.** The device of any preceding claim further including a printed circuit board electrically connected to a plurality of said contact points.

**6.** The device of any preceding claim, further including an antenna configured to transmit signals from the plurality of electrodes to a remote receiver.

**7.** The device of any preceding claim further including a third layer formed on the second layer, the conductive tracks and contact points being encapsulated by the third layer such that the conductive tracks are not accessible through the third layer, but the contact points are accessible.

**8.** The device of any preceding claim wherein the vias, tracks and contact points are formed of silver or a silver-based substance, graphite, copper, gold, a conductive polymer or composites of these materials.

**9.** The device of any preceding claim wherein the area covered by the first array is at least two times the area covered by the second array.

**10.** A method of fabricating a wearable device, the method including the steps of:

> forming a plurality of conductive electrodes on a textile layer, wherein the electrodes extend from a first side of the textile layer to a second, opposite, side;
> forming a second, insulating layer on the second side of the textile layer, overlaying the electrodes;
> forming through-holes in the second layer at the locations of the electrodes; applying conductive material to the through-holes to form conductive vias connected to the electrodes, the plurality of conductive vias defining a first array; and
> forming a plurality of tracks on the second layer, each track connecting one of the conductive vias to a contact point distal from the conductive via, the plurality of contact points forming a second array,
> wherein the area covered by the second array is smaller than the area covered by the first array.

**11.** The method of claim 10, further including the step of forming a third layer over the tracks such that the conductive tracks are encapsulated between the second and third layers, but the contact points are accessible through the third layer.

**12.** The method of any of claims 10-11 further including the step of connecting a printed circuit board to the contact points.

**13.** The method of any of claims 10-12 wherein the step of forming the electrodes uses blade-coating, screen-printing or other additive manufacturing techniques.

**14.** The method of any of claims 10-13 further including the step of forming a eutectogel coating on the electrodes on the first side of the textile layer.

Figure 1

Figure 2

Figure 3

Figure 4

Figure 5

# EP 4 674 347 A1

| | Europäisches Patentamt European Patent Office Office européen des brevets | **EUROPEAN SEARCH REPORT** | **Application Number** EP 24 38 6080 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 453 186 B1 (LOVEJOY DAVID ANTHONY [US] ET AL) 17 September 2002 (2002-09-17) * paragraphs [0024], [0029], [0029], [0031], [0037]; figures 1c,2i * | 1-14 | INV. A61B5/282 A61B5/00 |
| A | OWYEUNG RACHEL ET AL: "Eutectogel Electrodes for Long-Term Biosignal Monitoring", 2022 IEEE SENSORS, IEEE, 30 October 2022 (2022-10-30), pages 1-4, XP034243471, DOI: 10.1109/SENSORS52175.2022.9967321 [retrieved on 2022-12-08] * abstract * | 1,10,14 | |

|  | TECHNICAL FIELDS SEARCHED (IPC) |
|---|---|
|  | A61B |

The present search report has been drawn up for all claims

| Place of search | Date of completion of the search | Examiner |
|---|---|---|
| The Hague | 20 November 2024 | Knüpling, Moritz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 38 6080

This annex lists the patent family members relating to the patent documents cited in the above-mentioned European search report.
The members are as contained in the European Patent Office EDP file on
The European Patent Office is in no way liable for these particulars which are merely given for the purpose of information.

20-11-2024

| Patent document cited in search report | | Publication date | Patent family member(s) | | Publication date |
|---|---|---|---|---|---|
| US 6453186 | B1 | 17-09-2002 | EP | 1249204 A1 | 16-10-2002 |
| | | | JP | 2002325740 A | 12-11-2002 |
| | | | US | 6453186 B1 | 17-09-2002 |

EPO FORM P0459

For more details about this annex : see Official Journal of the European Patent Office, No. 12/82